(19) Europäisches Patentamt
European Patent Office
Office européen des brevets

(11) Publication number : 0 299 594 B1

# (12) EUROPEAN PATENT SPECIFICATION

(45) Date of publication of patent specification : **14.08.91 Bulletin 91/33**

(51) Int. Cl.$^5$ : **A61K 31/41**, A61K 31/19, A61K 31/10, // C07D257/04, C07D403/12, C07C321/02

(21) Application number : **88303569.3**

(22) Date of filing : **20.04.88**

## (54) Pharmaceutical use of aromatic thioethers.

(30) Priority : **22.04.87 GB 8709546**

(43) Date of publication of application : **18.01.89 Bulletin 89/03**

(45) Publication of the grant of the patent : **14.08.91 Bulletin 91/33**

(84) Designated Contracting States : **AT BE CH DE ES FR GB GR IT LI LU NL SE**

(56) References cited :
**EP-A- 0 186 426**
**EP-A- 0 190 042**
**EP-A- 0 228 045**
**GB-A- 2 144 422**

(73) Proprietor : **LILLY INDUSTRIES LIMITED**
**Kingsclere Road**
**Basingstoke Hants RG21 2XA (GB)**

(72) Inventor : **Baker, Stephen Richard**
**16 Whitley Road**
**Yateley Camberley Surrey (GB)**
Inventor : **Boot, John Robert**
**45 Beech Hill Road**
**Sunningdale Berkshire (GB)**

(74) Representative : **Hudson, Christopher Mark et al**
**Erl Wood Manor**
**Windlesham Surrey GU20 6PH (GB)**

EP 0 299 594 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of pharmaceutical compounds in the treatment of vascular diseases.

British Patent 2144422 discloses some compounds of the following formula

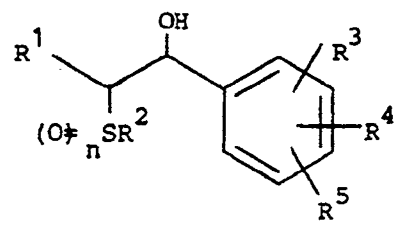

where n, $R^1$, $R^2$, $R^3$, $R^4$ and $R^5$ take various values. These compounds are described as useful in diseases such as for example allergic disorders.

We have now discovered that certain of these compounds are strongly indicated for use in the treatment of vascular diseases.

The invention comprises the use of a compound of the formula

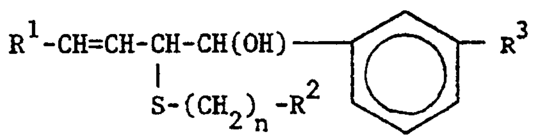

(I)

in which $R^1$ is $C_{7-20}$ alkyl or $C_{7-20}$ alkenyl containing 1 to 3 double bonds, the alkyl or alkenyl group being optionally substituted by phenyl, $R^2$ and $R^3$ are carboxyl or tetrazolyl, and n is 1 to 6 ; or a pharmaceutically-acceptable salt thereof ; for the manufacture of a medicament for the treatment of a vascular disease.

The compounds of formula (I) above and their pharmaceutically-acceptable salts are active in tests which indicate that they have potential in the treatment of cardiovascular diseases such as shock and ischaemic heart diseases for example coronary artery disease and myocardial infarction, cerebrovascular diseases, and renal diseases for example renal ischaemia.

In the above general formula, $R^1$ can be an alkyl group containing from 7 to 20 carbon atoms, and is preferably alkyl containing from 8 to 13 carbon atoms, such as for example 10 to 13 carbon atoms. The alkyl group is preferably straight chained. When $R^1$ is alkenyl it contains from 7 to 20 carbon atoms and preferably from 8 to 13 carbon atoms, such as for example 10 to 13 carbon atoms. Preferably the alkenyl group is unbranched and contains 1 or 3 double bonds. Particularly preferred alkenyl groups are those of formula $R^4CH{=}CH-$ where $R^4$ is $C_{6-11}$ alkyl such as $CH_3(CH_2)_m$ where m is 5 to 10, or $CH_3(CH_2)_4CH{=}CHCH_2CH{=}CH_2-$. When $R^1$ is substituted by phenyl, the phenyl group is preferably attached to the terminal carbon atom, for example, a group of the formula $PhCH_2(CH)_mCH{=}CH-$ where m is 5 to 10.

It will be appreciated that such double bonds, and the double bond between the 3 and 4 carbon atoms, provide opportunities for cis-trans isomeric forms. It is preferred that the $R^1$ group is arranged in trans configuration about the double bond at the 3 and 4 carbon atoms. It will also be appreciated that the compounds of formula (I) possess chiral centres at the carbon atoms bearing the hydroxyl and thio groups and, accordingly, stereoisomeric forms exist R, R ; S, S ; R, S ; and S, R. The use of all such stereoisomers, and racemic mixtures thereof, is included within the scope of the invention. The preferred compounds are of S, R configuration.

Examples of particularly preferred compounds for use in the invention are those in which the moiety $R^1CH{=}CH$ in formula (I) above is

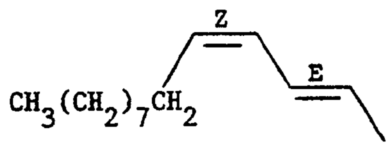

and

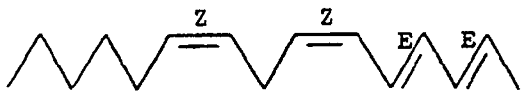

In formula (I) above the value of n in the formula can be from 1 to 6. It is preferably 1, 2 or 3 and in the most preferred compounds n is 2. The values of $R^2$ and $R^3$ can, of course, be different and $R^3$ is preferably tetrazolyl.

The compound of formula (I) bears two acidic functions and base addition salts can thus be prepared. The use of these salts is also included as part of the present invention. Examples of such salts are those derived from alkali and alkaline earth metal hydroxides, carbonates and bicarbonates, as well as salts derived from aliphatic and aromatic amines, aliphatic diamines and hydroxy alkylamines. The potassium and sodium salt forms are particularly preferred.

A particular group of compounds according to formula (I) for use in the treatment of vascular diseases, are those of the formula :

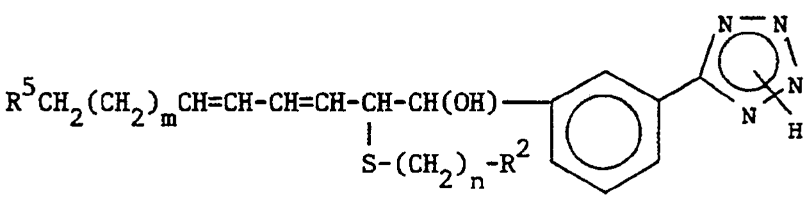

in which $R^5$ is hydrogen or phenyl, m is 5 to 10, n is 1, 2 or 3 and $R^2$ is carboxyl or tetrazolyl ; and pharmaceutically-acceptable salts thereof. A further preferred group is of the formula

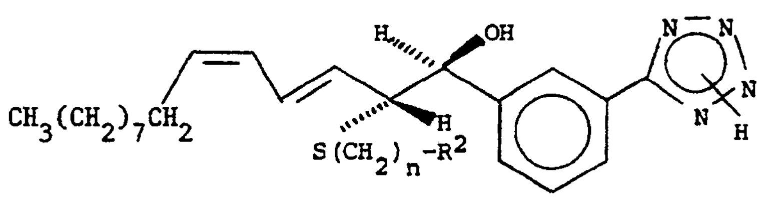

in which n is 1, 2 or 3 ; and pharmaceutically-acceptable salts thereof. Such compounds exhibit 1S, 2R chirality.

Specific examples of compounds for use in the invention are :

(1S,2R)-5-{3-[2-(2-Carboxyethylthio)-1-hydroxypentadeca-3(E), 5(Z)-dienyl]phenyl}-1H-tetrazole,
(1S,2R)-5-{3-[2-(2-1H-tetrazol-5-ylethylthio)-1-hydroxypentadeca-3(E),5(Z)-dienyl]phenyl}-1H-tetrazole,
(1S,2R)-5-{3-[2-(2-carboxyethylthio)-1-hydroxy-13-phenyltrideca-3(E),5(Z)-dienyl]phenyl}-1H-tetrazole,
(1S,2R)-5-{3-[2-1H-tetrazol-5-ylethylthio)-1-hydroxy-17-phenylheptadeca-3(E),5(Z) -dienyl]phenyl}-1H-tetrazole,
(1S,2R)-3-[2-(2-carboxyethylthio)-1-hydroxypentadeca-3(E),5(Z)-dienyl]benzoic acid,
(1S,2R)-5-{3-[2-1H-tetrazol-5-ylethylthio)-1-hydroxy-13-phenyltrideca-3(E),5(Z)-dienyl]phenyl}-1H-tetrazole,
(1S,2R)-3-[2-(2-carboxyethylthio)-1-hydroxy-16-phenylhexadeca-3(E),5(Z),13(Z)-trienyl]benzoic acid,
(1S,2R)-3-[2-(2-carboxyethylthio)-1-hydroxy-16-phenylhexadeca- 3(E),5(Z)-dienyl]benzoic acid ;

and their pharmaceutical salts.

The compounds of formula (I) can be prepared by methods which are fully described in British Patent 2144422 and British Patent Application 2168704. The first two compounds referred to above are, for example, disclosed in Example 45 of British Patent 2144422 and Example 1 of British Patent Application 2168704, respectively.

The pharmaceutical properties of the compounds of formula (I) and their potential in the treatment of vascular diseases have been demonstrated in the test described by Shipley R.E. and Tilden J.H. Proc. Soc. exp. Biol. Med. 64, 453-455 (1947). This test employs the pithed rat as model and measures the effect of a compound in restoring blood pressure after challenge with $LTD_4$. The following list gives the $ED_{50}$ value in this test for the named compounds :

(1S,2R)-5-{3-[2-(2-1H-tetrazol-5-ylethylthio)-1-hydroxypentadeca-3(E),5(Z)-dienyl]phenyl}-1H-tetrazole, 0.7 mg/kg I.V.

(1S,2R)-5-{3-[2-(2-carboxyethylthio)-1-hydroxy-13-phenyltrideca-3(E),5(Z)-dienyl]phenyl}-1H-tetrazole, 3.6 mg/kg I.V.

(1S,2R)-5-{3-[2-1H-tetrazol-5-ylethylthio)-1-hydroxy-17-phenylheptadeca-3(E), 5(Z) -dienyl]phenyl}-1H-tetrazole, 2.2 mg/kg I.V.

(1S,2R)-3-[2-(2-carboxyethylthio)-1-hydroxypentadeca-3(E),5(Z)-dienyl]benzoic acid, 9.6 mg/kg I.V.

The compounds may be administered by various routes, for examples by the oral or rectal route, topically, by inhalation, and especially parenterally, for example by intravenous injection or infusion. They are usually employed in the form of a pharmaceutical composition. Such compositions are prepared in a manner well known in the pharmaceutical art and normally comprise at least one active compound. In making the compositions, the active ingredient will usually be in salt form mixed with a carrier, or diluted by a carrier, which may be a solid, semi-solid, or liquid material which acts as a vehicle, excipient or medium for the active ingredient. Thus, the composition may be in the form of injection solutions, suspensions, ointments, tablets, lozenges, sachets, cachets, elixirs, aerosols (as a solid or in a liquid medium), soft and hard gelatin capsules, suppositories, and sterile packaged powders.

For preferred, parenteral, administration forms of presentation include injectible solutions and suspensions, and infusions. Injectible solutions preferably comprise a complexing agent for example a modified starch such as β-cyclodextrin or a protein such as human serum albumin, or a lecithin. For example a typical 5 ml injection contains 1 to 5 per cent of active compound and 5 to 30 per cent of β-cyclodextrin in 0.9 per cent saline solution. A typical infusion (500 ml) contains 0.01 to 1 per cent of active compound and 0.01 to 1 per cent sodium bicarbonate in 5 per cent dextrose solution.

When compositions for use according to the invention are formulated in unit dosage form, it is preferred that each unit dosage form contains from 1 mg to 500 mg, for example, from 5 mg to 100 mg.

The active compounds are effective over a wide dosage range and, for example, dosages per day will normally fall within the range of from 0.01 to 50 mg/kg, more usually in the range of from 0.05 to 10 mg/kg. However, it will be understood that the amount administered will be determined by the physician in the light of the relevant circumstances including the condition to be treated, the choice of compound to be administered and the chosen route of administration, and therefore the above dosage ranges are not intended to limit the scope of the invention in any way.

## Claims

1. Use of a compound of the formula

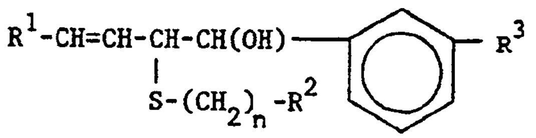

(I)

in which $R^1$ is $C_{7-20}$ alkyl or $C_{7-20}$ alkenyl containing 1 to 3 double bonds, the alkyl or alkenyl group being optionally substituted by phenyl, $R^2$ and $R^3$ are carboxyl or tetrazolyl, and n is 1 to 6 ; or a pharmaceutically-acceptable salt thereof ; for the manufacture of a medicament for the treatment of a vascular disease.

2. Use of a compound according to claim 1, in which $R^1$ is $R^4CH{=}CH$— where $R^4$ is $C_{6-11}$ alkyl.

3. Use of a compound according to either of claims 1 and 2, in which n is 2 and $R^3$ is tetrazolyl.

4. Use of a compound according to claim 1, of the formula

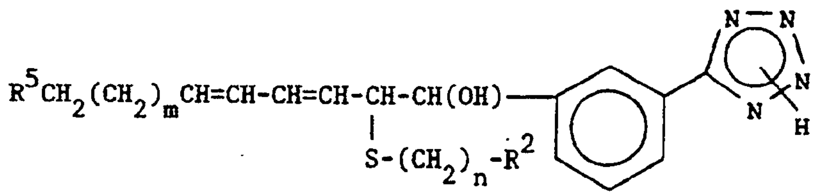

in which $R^5$ is hydrogen or phenyl, m is 5 to 10, n is 1, 2 or 3 and $R^2$ is carboxyl or tetrazolyl ; and pharmaceutically-acceptable salts thereof.

5. Use of a compound according to claim 4 of the formula

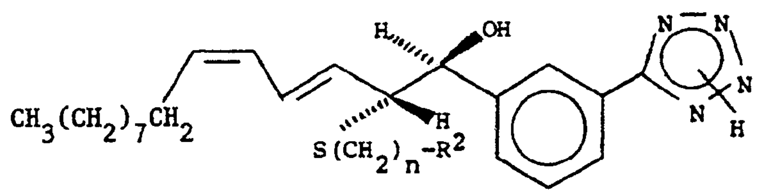

in which $R^2$ is carboxyl or tetrazolyl and n is 1, 2 or 3, or a pharmaceutically-acceptable salt thereof.

**Patentansprüche**

1. Verwendung einer Verbindung der Formel

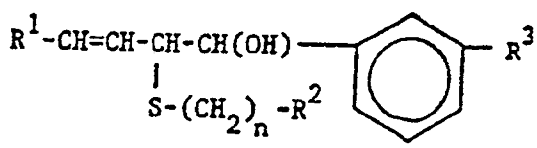

(I)

worin $R^1$ $C_{7-20}$ Alkyl oder $C_{7-20}$ Alkenyl mit 1 bis 3 Doppelbindungen ist, wobei die Alkyl- oder Alkenylgruppe gegebenenfalls durch Phenyl substituiert ist, $R^2$ und $R^3$ Carboxyl oder Tetrazolyl sind, und n 1 bis 6 ist ; oder eines pharmazeutisch brauchbaren Salzes davon ; zur Herstellung eines Arzneimittels zur Behandlung einer vaskulären Erkrankung.

2. Verwendung einer Verbindung nach Anspruch 1, worin $R^1$ $R^4CH{=}CH$— ist, worin $R^4$ $C_{6-11}$ Alkyl ist.

3. Verwendung einer Verbindung nach einem der Ansprüche 1 und 2, worin n 2 ist und $R^3$ Tetrazolyl ist.

4. Verwendung einer Verbindung nach Anspruch 1, mit der Formel

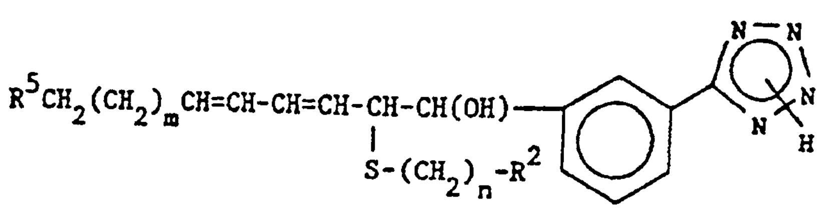

worin $R^5$ Wasserstoff oder Phenyl ist, m 5 bis 10 ist, n 1, 2 oder 3 ist und $R^2$ Carboxyl oder Tetrazolyl ist ; und

von pharmazeutisch brauchbaren Salzen davon.

5. Verwendung einer Verbindung nach Anspruch 4 mit der Formel

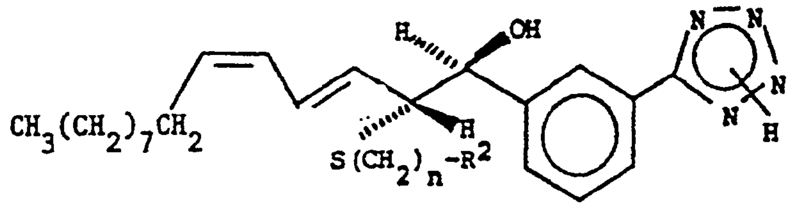

worin $R^2$ Carboxyl oder Tetrazolyl ist und n 1, 2 oder 3 ist, oder eines pharmazeutisch brauchbaren Salzes davon.

## Revendications

1. Utilisation d'un composé de formule

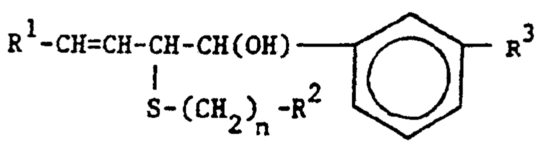

(I)

dans laquelle $R^1$ représente un groupe alkyle en $C_7$-$C_{20}$ ou un groupe alcényle en $C_7$-$C_{20}$ contenant de 1 à 3 doubles liaisons, le groupe alkyle ou le groupe alcényle étant éventuellement substitué par un groupe phényle, $R^2$ et $R^3$ représentent un groupe carboxyle ou un groupe tétrazolyle et n est égal à 1-6, ou encore d'un de ses sels pharmaceutiquement acceptables, pour la préparation d'un médicament destiné au traitement d'une maladie vasculaire.

2. Utilisation d'un composé selon la revendication 1, dans lequel $R^1$ représente $R^4CH{=}CH$— où $R^4$ représente un groupe alkyle en $C_6$-$C_{11}$.

3. Utilisation d'un composé selon l'une quelconque des revendications 1 et 2, dans lequel n est égal à 2 et $R^3$ représente un groupe tétrazolyle.

4. Utilisation d'un composé selon la revendication 1, répondant à la formule

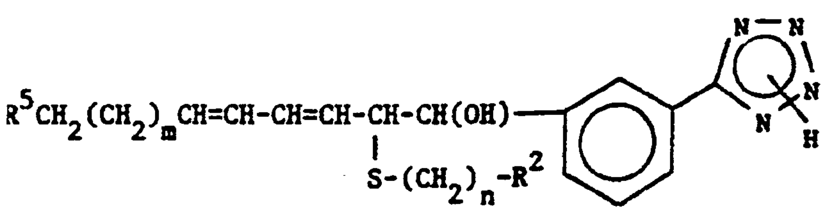

dans laquelle $R^5$ représente un atome d'hydrogène ou un groupe phényle, m est égal à 5-10, n est égal à 1, 2 ou 3 et $R^2$ représente un groupe carboxyle ou un groupe tétrazolyle, ainsi que d'un de ses sels pharmaceutiquement acceptables.

5. Utilisation d'un composé selon la revendication 4, répondant à la formule

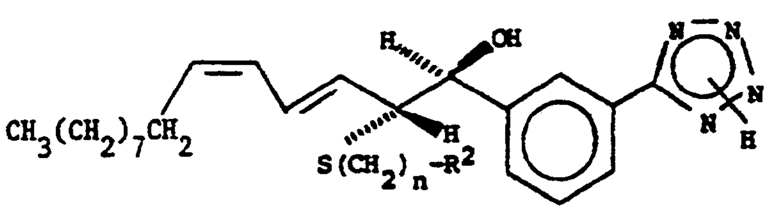

dans laquelle $R^2$ représente un groupe carboxyle ou un groupe tétrazolyle et n est égal à 1, 2 ou 3, ou d'un de

ses sels pharmaceutiquement acceptables.